# EUROPEAN PATENT SPECIFICATION

(11) **EP 0 620 007 B1**
(45) Date of publication and mention of the grant of the patent: **01.09.1999**
(21) Application number: 94301102.3
(22) Date of filing: 16.02.1994
(51) Int. Cl.: A61K 35/78

(54) **Anti-ulcer agent**
Antigeschwürmittel
Agent contre les ulcéres

(30) Priority: 16.02.1993 JP 4878593; 28.12.1993 JP 34940693
(43) Date of publication of application: 19.10.1994
(73) Proprietor: SOKEN Co., Ltd., Ayauta Gun, Kagawa Ken (JP)
(72) Inventor: Tokuyama, Takashi, Ayauta-Gun, Kagawa-Ken (JP)
(74) Representative: Dixon, Donald Cossar

(56) References cited:
- EP-A- 0 027 514
- EP-A- 0 542 398
- DATABASE WPI Section Ch, Week 9237, Derwent Publications Ltd., London, GB; Class B04, AN 92-304664 & JP-A-4 210 645 (SO-KEN KK) 31 July 1992
- PATENT ABSTRACTS OF JAPAN vol. 015, no. 409 (C-0876)18 October 1991 & JP-A-03 168 054 (MITSUO KODA) 19 July 1991
- DATABASE WPI Section Ch, Week 9213, Derwent Publications Ltd., London, GB; Class B04, AN 92-102783 & JP-A-4 049 240 (CHIBA SEIFUN KK) 18 February 1992

## Description

The present invention relates to an anti-ulcer agent obtained from germinated rice and having activity useful for both the prophylaxis and treatment of ulcers via oral or subcutaneous administration.

Uses of rice which have been hitherto developed include 'sake', 'shochu' (Japanese spirits distilled from rice), vinegar and 'koji' (malted rice), as well as use as a staple food and, thus, rice has long been indispensable in people's lives. In addition, the use of a small rice-bran bag for cosmetic purposes has also been known. Thus rice has previously been considered only as a simple staple food or as a starch source, and there has been neither the conception that rice has a therapeutically effective component, nor that this effective component can be usefully exploited.

Current daily life involves much stress and the chances of suffering such stress have been increased both by kaleidoscopic changes in the daily environment, as well as an increase in the complexity of personal relationships. Also, the chance of taking many victuals that do not exist in nature has been increased.

In this way, the number of persons suffering from, for example, a stomach ulcer or duodenal ulcer, because of these factors has increased, and various anti-ulcer agents have been developed and are being utilized. The anti-ulcer agents that have been used to date can be largely classified into, for example, digestive power depressants, gastric juice secretion depressants, and mucous membrane protective tissue reparative agents, and are orally or subcutaneously administered. However, as these preparations are isolated or synthesized medicaments, and each medicament has side effects, the restrictions on the indications for employment and the dosage become severe. Thus, an effective and safe anti-ulcer agent has not yet been developed and utilized.

Since these conventional anti-ulcer agents cannot be regularly used from the point of safety, they cannot be utilized for either prophylaxis or recurrence prevention. On the other hand, as preventatives for ulcers, there have been used only medicines for intestinal disorders and medicaments having the effect of preventing secretion of gastric juices, and these cannot be said to be true ulcer preventatives.

Currently,the side effects of medicaments on humans have become a problem and hence there has been required the development of a medicament which has an anti-ulcer effect, is a natural product, has no side effects, and is sufficiently safe, even when the medicament is regularly used as a preventative or as a recurrence-prevention agent.

The inventor has already developed an anti-ulcer agent as disclosed in unexamined published Japanese Patent Application No 4-210645 (210645/1962). However, the development of an anti-ulcer agent which can be prepared more inexpensively and more easily, and which has an anti-ulcer effect that is the same as, or better than, that of the foregoing previous invention, has not been achieved.

The object of the present invention is, therefore, to provide a natural anti-ulcer agent obtained from germinated rice, which has excellent anti-ulcer effects, and is inexpensive and safe even when the medicament is regularly used as a preventative or a recurrence prevention agent for ulcers.

The inventor has investigated various vegetable components centring around rice which is the staple food from the view point of the environmental harmonization of animals and plants. It has become apparent that rice has many possibilities and effects which have never been anticipated until now. Rice, which has been used as a staple food and the safety of which has thus been actually proved, has been adopted as the subject of various investigations into its general utilization, and in particular into anti-ulcer agents obtained from rice. During the course of these investigations, it has been discovered that rice and germinated rice each contain a component exhibiting a very remarkable anti-ulcer activity in the cases of both oral and subcutaneous administration, and the present invention has been based on this discovery.

The component having the anti-ulcer effect and contained germinated rice has not yet been identified, but it has been confirmed that rice and germinated rice in the forms described below exhibit an anti-ulcer effect:-
(1) The ground product of germinated rice itself or a material containing the ground product.
(2) An extract of germinated rice or a material containing the extract.
(3) The product obtained by effecting enzymatic decomposition of, or by adding koji (malted rice) to, the hydrated product of rice or of germinated rice, or a material containing the foregoing product.
(4) The product obtained by extracting germinated rice, the product being subjected to enzymatic decomposition or the addition of koji before extraction, during extraction or after extraction, or a material containing the foregoing product.
(5) The product obtained by the alcohol-fermentation or organic acid-fermentation of an extract of germinated rice or by the alcohol-fermentation or organic acid-fermentation of the product obtained by subjecting rice or germinated rice to enzymatic decomposition or 'koji' treatment, or a material containing the fermentation product, to an enzyme decomposition or koji.

The present invention will now be described in detail.

In the present invention the term "rice" means any rice harvested not only in Japan but also in other countries; Germinated rice is used in the present invention.

In addition, since the effective component of germinated rice is stable to heat and light, the raw materials described above may be subjected to a surface denaturation such as dipping, cooking, broiling (including sand broiling, netting broiling and hot blast broiling), cook broiling and lyophilization; light denaturation such as an UV irradiation; press broiling such as pat rice; or a raw material treatment such as frying. Also, the effect of the components is not changed by applying thereto the foregoing treatments.

Germinated rice can be used effectively as it is; however, from the view point of practical use, it is preferred to use them as the ground products. For powdering germinated rice by grinding, a general method using a grinder or a rice-cleaning machine may be used.

To germinate rice, rice with embryo buds is immersed in water or water is sprayed onto rice with embryo buds in order to cause it to germinate. The germination temperature is from 5°C to 70°C. However, if rice germinates, there are no restrictions on the temperature and the time of germination. Also, if there is a possibility of the water putrefying during the germination, it is preferred to renew the water to prevent it from putrefying or to apply an antiseptic agent.

In this invention, the term "germination" means any stage of the germination process of rice from directly before germination to fully germinated rice. Germinated rice is used after being washed well and in this case, the washed germinated rice may be dried.

If, in the case of the extraction of germinated rice, or during the enzymatic decomposition or 'koji' treatment of rice, or germinated rice, the raw material rice, or germinated rice, is ground into a granular form or a powder form, the surface area of the raw material is increased to improve efficiency. The raw material need not be ground, but in this case a long time is required for the decomposition or the extraction of the rice tissue.

In the case of extracting germinated rice with water, efficiency is improved by increasing the extraction temperature, but even at a low temperature the extraction can be carried out sufficiently. However, when the extraction temperature is lower than 40°C, it is preferable that the pH of the system be adjusted to an acidic or an alkaline range, or than an antiseptic or an alcohol be added to the system to prevent the germinated rice from rotting.

The extraction time may be long or short providing that the effective component is extracted, and may be determined according to the extraction temperature. Also, the extraction may be carried out under increased pressure, at normal pressure or under reduced pressure.

In the case of the water extraction of germinated rice, the most troublesome problem is the phenomenon of gelatinization. If rice is gelatinized, not only is the extraction efficiency reduced but also the practical extraction operation becomes very difficult. To prevent the occurrence of gelatinization of the rice, the extraction may be carried out with the addition of amylase or the system may be acidified with hydrochloric acid to cut the starch. By employing the foregoing method the problem can be solved adequately, and there is no practical problem is encountered in employing the method.

Since the effective component in the extracted product may be stable in the presence of an acid or an alkali, acidic decomposition extraction or alkaline decomposition extraction can be carried out. In this case, if necessary, neutralization and desalting can be performed subsequently.

Even in the case of extraction with an organic solvent, it is desirable to grind or powder the rice or powder the rice or germinated rice as fine as possible during extraction. As the organic solvent, a general organic solvent such as, for example, ethanol, acetone, n-hexane or methanol, can be used but since any organic solvent which is noxious to humans must be completely removed from the extract, the use of an organic solvent that is not noxious to humans is preferable.

Also, the germinated rice may be subject to an enzymatic decomposition or 'koji' treatment. The term "enzyme decomposition" in this invention means that one or more kinds of enzymes are caused to act on the rice or germinated rice, such as an amylolytic enzyme, a proteolytic enzyme, lipase, a fibre decomposition enzyme, a lignin decomposition enzyme or a pectin decomposition enzyme.

Also, there are no restrictions on the type of 'koji' moulds or the strain and kind of rice used for producing 'koji'. Furthermore, when carrying out the extraction described above, the foregoing enzyme decomposition or 'koji' treatment may be performed before, during or after the extraction.

In the present invention, when an alcohol fermentation or an organic acid fermentation such as, for example, a lactic acid fermentation or an acetic acid fermentation, is applied simultaneously with or after the treatment described above, the treatment becomes more effective as described below.

First, when alcohol fermentation is applied, concentration of the treated product is facilitated, enabling the effective component to be easily concentrated. Also, lactic acid fermentation improves the flavour of the product in the case of using the product in, for example, drinks and by applying the acetic acid fermentation, the product can be utilized as a seasoning liquid such as vinegar. Thus, by utilizing organic acid fermentation, the product finds wide application.

The product of the present invention obtained as described above may be used as it is without separation of residues or may be used after compression and/or filtration. When the product is used as it is, it should be subjected to sterilization or removal of fungi. In addition, the product may be dried and used, for example, as granules or tablets. Furthermore, the product can be used after compounding with various foods.

Next, the anti-ulcer effects of the products of the present invention will be described below.

### Prophylaxis effect on the occurrence of stress ulcer:

To determine the effect of the product of the present invention as an anti-ulcer agent, its effect on a restrained water-immersed stress ulcer by oral administration was investigated. The method was carried out according to the Watanabe et al method, as follows:

After fasting ddY series male mice aged 8 weeks for 24 hours, the product obtained in each of the Examples described hereinafter was orally administered to the mice at 0.3 ml/mouse. After 30 minutes, the mice were placed in a stress gauge, and were immersed in water at 15°C up to the xiphisternum of each mouse, thereby applying a restrained water-immersed stress. After 5 hours, each mouse was slaughtered by luxating the cervical vertebrae thereof and the stomach was extracted. Thereafter, 1.5 ml of a 1% formalin solution was infused into the stomach followed by immersing the stomach in the solution, whereby the stomach tissue was lightly fixed, and the stomach was allowed to stand as it was for 24 hours. Thereafter, the stomach was incised along the curvatura ventriculi major, the length (mm) of each injury formed at the glandular stomach was measured, and the sum total thereof per one mouse was shown as the ulcer coefficient.

Also, as a control, mice were orally administered with the same amount of an isotonic sodium chloride solution 30 minutes before being placed in the stress gauge.

For each test, 15 mice were used. The results obtained are shown in Table 1 below.

**Table 1**

| Product Obtained in Example | Doses | No. of Test Mice | Average Ulcer Coefficient |
|---|---|---|---|
| Example 1 | 0.3 g | 15 | 10.4 |
| Example 2 | 0.3 ml | 15 | 6.3 |
| Example 4 | 0.3 ml | 15 | 2.2 |
| Example 4* | 0.3 ml | 15 | 2.1 |
| Example 5 | 0.3 ml | 15 | 0.9 |
| Example 6 | 0.3 ml | 15 | 3.1 |
| Example 8 | 0.3 ml | 15 | 2.7 |
| Example 10 | 0.3 ml | 15 | 7.1 |
| Example 12 | 0.3 ml | 15 | 5.1 |
| Example 14 | 0.3 ml | 15 | 5.4 |
| Example 16 | 0.3 ml | 15 | 4.7 |
| Example 18 | 0.3 ml | 15 | 3.8 |
| Example 20 | 0.3 ml | 15 | 5.6 |
| Example 22 | 0.3 ml | 15 | 2.8 |
| Example 24 | 0.3 ml | 15 | 1.0 |
| Example 26 | 0.3 ml | 15 | 1.6 |
| Example 28 | 0.3 ml | 15 | 1.4 |
| Example 30 | 0.3 ml | 15 | 0.2 |
| Example 32 | 0.3 ml | 15 | 0.4 |
| Example 34 | 0.3 ml | 15 | 0.7 |
| Control** | 0.3 ml | 15 | 25.2 |

| | | | |
|---|---|---|---|
| (*): Product obtained as in Example 4 using glutinous rice. | | | |
| (**): Isotonic sodium chloride solution was used. | | | |

As shown in Table 1, it can be seen that while the average ulcer coefficient in the mice administered with an isotonic sodium chloride solution as a control was 25.2, the average ulcer coefficient in the mice administered with the products of the present invention was very low in all the cases. This clearly shows that the oral administration of the product of the present invention is effective as an anti-ulcer agent for restrained water-immersion stress ulcers.

As a result, it has been confirmed that the product of the present invention directly acts from the mucous membrane of the stomach and intestines to exhibit an effective activity as an anti-ulcer agent.

In addition, when the prophylactic effect of the rice bran extract, obtained by processing rice bran formed by rice cleaning as in Example 4, was also tested as described above, not only did the extract show no effect but also there was rather a tendency to increase the ulcer coefficient.

Next, the effect of the products of this invention on a restrained water-immersion stress ulcer by subcutaneous administration was investigated. This method was carried out according to the Watanabe et al method as for the oral administration described above.

15 mice (control) subcutaneously administered with 0.3 ml of an isotonic sodium solution and 15 mice subcutaneously administered with the product of the present invention obtained in each of the Examples, were placed in a stress gauge after having been allowed to stand for 30 minutes. A restrained water-immersion stress was applied to the mice, and the effectiveness of subcutaneous administration of the products of the present invention on restrained water-immersion stress ulcer was determined. The results are shown in Table 2 below.

**Table 2**

| Product Obtained in Example | Doses | No. of Test Mice | Average Ulcer Coefficient |
|---|---|---|---|
| Example 2 | 0.3 ml | 15 | 8.7 |
| Example 4 | 0.3 ml | 15 | 2.1 |
| Example 4* | 0.3 ml | 15 | 2.4 |
| Example 5 | 0.3 ml | 15 | 1.1 |
| Example 6 | 0.3 ml | 15 | 3.4 |
| Example 8 | 0.3 ml | 15 | 3.0 |
| Example 10 | 0.3 ml | 15 | 7.5 |
| Example 12 | 0.3 ml | 15 | 5.6 |
| Example 14 | 0.3 ml | 15 | 5.4 |
| Example 16 | 0.3 ml | 15 | 5.7 |
| Example 18 | 0.3 ml | 15 | 4.8 |
| Example 20 | 0.3 ml | 15 | 5.4 |
| Example 22 | 0.3 ml | 15 | 3.1 |
| Example 24 | 0.3 ml | 15 | 12 |
| Example 26 | 0.3 ml | 15 | 18 |
| Example 28 | 0.3 ml | 15 | 15 |
| Example 30 | 0.3 ml | 15 | 0.3 |
| Example 32 | 0.3 ml | 15 | 0.5 |
| Example 34 | 0.3 ml | 15 | 0.7 |
| Control** | 0.3 ml | 15 | 26.1 |

| | | | |
|---|---|---|---|
| (*), (**): Same as in Table 1 described above. | | | |

As shown in Table 2, it can be seen that while the average ulcer coefficient in the mice subcutaneously administered with 0.3 ml of an isotonic sodium chloride solution was 26.1, the average ulcer coefficient of the mice subcutaneously administered with 0.3 ml of each product of the present invention was very low in all cases. This result which shows that the subcutaneous administration of the product of this invention is effective as an anti-ulcer treatment.

The foregoing fact that the product of the present invention exhibits an effective anti-ulcer activity as an anti-ulcer agent in a subcutaneous administration as described above proves that, not only does the product of the present invention have a direct effect on mucous membrane, but also that the product has an effective component capable of fundamentally preventing the occurrence of stomach ulcers through the blood.

From the results described above, it has been confirmed that the product of the present invention is effective for prophylaxis of the occurrence of stress ulcers based on a component which is effective via both oral and subcutaneous administration.

Next, the treatment effects of the products of the present invention on stomach ulcers of mice were investigated.

Hitherto, in the determination of the treatment effects on ulcers using rats, there has been used either (1) a 'shochu' gastrin ulcer, or (2) an acetic acid ulcer. On the other hand, in the present invention a method capable of easily determining ulcer treating effects using mice has been developed and has been used for determining such effects on stomach ulcers of mice. The method is as follows.

After fasting ddY series male mice for 24 hours, the mice were placed in a stress gauge and immersed in water of 15°C up to the xiphisternum of each mouse to apply a restrained water-immersed stress. After 5 hours, 0.3 ml of the product of the present invention was immediately orally administered to each mouse. After another 2 hours, each mouse was slaughtered by luxating the cervical vertebrae, and the stomach was extracted. Thereafter, 1.5 ml of a 1% formalin solution was infused into the stomach, the stomach was further immersed in the solution, whereby the stomach was lightly fixed, and thereafter, the ulcer coefficient was measured.

Also, as a control, mice were orally administered with an isotonic sodium chloride solution.

In the test, 15 mice were used in each case.

The results obtained are shown in Table 3 below.

**Table 3**

| Product Obtained in Example | Doses | No. of Test Mice | Average Ulcer Coefficient |
|---|---|---|---|
| Example 1 | 0.3 g | 15 | 11.5 |
| Example 2 | 0.3 ml | 15 | 14.8 |
| Example 4 | 0.3 ml | 15 | 2.4 |
| Example 4* | 0.3 ml | 15 | 2.1 |
| Example 5 | 0.3 ml | 15 | 4.5 |
| Example 6 | 0.3 ml | 15 | 3.8 |
| Example 8 | 0.3 ml | 15 | 3.2 |
| Example 10 | 0.3 ml | 15 | 7.7 |
| Example 12 | 0.3 ml | 15 | 5.7 |
| Example 14 | 0.3 ml | 15 | 5.4 |
| Example 16 | 0.3 ml | 15 | 6.3 |
| Example 18 | 0.3 ml | 15 | 5.2 |
| Example 20 | 0.3 ml | 15 | 5.9 |
| Example 22 | 0.3 ml | 15 | 4.2 |
| Example 24 | 0.3 ml | 15 | 1.5 |
| Example 26 | 0.3 ml | 15 | 2.2 |
| Example 28 | 0.3 ml | 15 | 2.0 |
| Example 30 | 0.3 ml | 15 | 0.9 |
| Example 32 | 0.3 ml | 15 | 1.2 |
| Example 34 | 0.3 ml | 15 | 1.3 |
| Control** | 0.3 ml | 15 | 27.2 |

| | | | |
|---|---|---|---|
| (*), (**): Same as in Table 1. | | | |

From the results for the average ulcer coefficients shown in the above Table, it can be seen that in contract to the case of orally administering an isotonic sodium chloride solution, the effect on stomach ulcer is clearly effective in all cases of orally administering the products of the present invention.

The present invention is next described further in the following Examples.

### Example 1

By immersing 1 kg of rice with germs in water of 25°C for 3 days, rice was germinated. The germinated rice, after washing well, was dried at 50°C for 24 hours and finely ground to provide 990 g of the product of the present invention

### Comparative Example 2

Polished rice was ground in a grinder to provide 500 g of the ground product. To the ground product was added 1,500 ml of water and, after lowering pH thereof with hydrochloric acid, the mixture was allowed to stand for 10 days. Thereafter, the mixture was squeezed by a squeezer and the clear liquid obtained was neutralized to provide 1,200 ml of the product of the present invention and 760 g of residues.

### Example 3

A further 1,190 ml of product of the present invention was obtained by following the same procedure as in Example 2 using 500 g of the product obtained in Example 1.

### Comparative Example 4

Polished rice was ground in a grinder to provide 500 g of the ground product. To the ground product were added 10 g of a liquefied enzyme and 1,500 ml of water. Thereafter, the temperature of the mixture was gradually raised and after extracting under boiling for 5 minutes, the extract was cooled. Then, the extract was squeezed by a squeezer to provide 1,420 ml of the product of the present invention and 560 g of residues.

### Example 5

A further 1,400 ml of product of the present invention was obtained by following the same procedure as in Example 4 using 500 g of the product obtained in Example 1.

### Comparative Example 6

Polished rice was ground in a grinder to provide 500 g of the ground product. To the ground product was added 1,500 ml of an aqueous 2N sodium hydroxide solution and the resultant mixture was allowed to stand for 5 days. Thereafter, the mixture was squeezed by a squeezer to provide 1,350 ml of a clear liquid and 650 g of residues. By neutralizing the clear liquid with 10N hydrochloric acid, 1,480 ml of the product of the present invention was obtained.

### Example 7

A further 1,490 ml of product of the present invention was obtained by following the same procedure as in Example 6 using 500 g of the product obtained in Example 1.

### Comparative Example 8

Polished rice was ground in a grinder to provide 500 g of the ground product. To the ground product was added 1,500 ml of 95% ethanol and the mixture was allowed to stand for 5 days. Thereafter, the mixture was squeezed by a squeezer to provide 1,300 ml of the clear liquid and 650 g of residues. To the clear liquid was added 2,000 ml of water and the mixed liquid was concentrated by a rotary evaporator to provide 1,500 ml of the product of the present invention.

### Example 9

A further 1,500 ml of product of the present invention was obtained by following the same procedure as in Example 8 using 500 g of the product obtained in Example 1.

### Comparative Example 10

Polished rice was ground in a grinder to provide 500 g of ground product. To the ground product were added 300 g of 'koji' and 1,500 ml of water and the mixture was allowed to stand for 20 hours at 55°C. Thereafter, the mixture was squeezed in a squeezer to provide 1,230 ml of the product of the present invention and 1,000 g of residues.

### Example 11

A further 1,230 ml of product of the present invention was obtained by following the same procedure as Example 10 using 500 g of the product obtained in Example 1.

### Comparative Example 12

Polished rice was ground by a grinder to provide 500 g of ground product. To the ground product were added 2 g of proteolytic enzyme and 1,500 ml of water and the resultant mixture was allowed to stand at 50°C for 2 hours. Thereafter, the mixture was squeezed by a squeezer to provide 1,310 ml of the product of the present invention and 670 g of residues.

### Example 13

A further 1,380 ml of product of the present invention was obtained by following the same procedure as in Example 12 using 500 g of the product obtained in Example 1.

### Comparative Example 14

Polished rice was ground in a grinder to provide 500 g of the ground product. To the ground product were added 2 g of lipase and 1,500 ml of water and the resultant mixture was allowed to stand at 50°C for 20 hours. Thereafter, the mixture was squeezed by a squeezer to provide 1,290 ml of the product of the present invention and 680 g of residues.

### Example 15

A further 1,360 ml of the product of the present invention was obtained by following the same procedure as in Example 14 using 500 g of the product obtained in Example 1.

### Comparative Example 16

Polished rice was ground in a grinder to provide 500 g of the ground product of polished rice. To the ground product were added 2 g of a fibre decomposition enzyme and 1,500 ml of water and the resultant mixture was allowed to stand at 50°C for 20 hours. Thereafter, the mixture was squeezed by a squeezer to provide 1,330 ml of the product of the present invention and 650 g of residues.

### Example 17

A further 1,370 g of product of the present invention was obtained by following the same procedure as in Example 16 using 500 g of the product obtained in Example 1.

### Comparative Example 18

Polished rice was ground in a grinder to provide 500 g of the ground product. To the ground product were added 2 g of an amylolytic enzyme and 1,500 ml of water and the resultant mixture was allowed to stand at 55°C for 20 hours. Thereafter, the mixture was squeezed by a squeezer to provide 1,380 ml of the product of the present invention and 600 g of residues.

### Example 19

A further 1,400 ml of product of the present invention was obtained by following the same procedure as in Example 18 using 500 g of the product obtained in Example 1.

### Comparative Example 20

Polished rice was ground in a grinder to provide 500 g of the ground product. To the ground product were added 2 g of a pectin decomposition enzyme and 1,500 ml of water and the resultant mixture was allowed to stand at 50°C for 20 hours. Thereafter, the mixture was squeezed by a squeezer to provide 1,320 ml of the product of the present invention and 660 g of residues.

### Example 21

A further 1,300 ml of product of the present invention was obtained by following the same procedure as in Example 20 using 500 g of the product obtained in Example 1.

### Comparative Example 22

Polished rice was ground in a grinder to provide 500 g of the ground product of polished rice. To the ground product were added 2 g of a proteolytic enzyme, 2 g of lipase, 2 g of a fibre decomposition enzyme, 2 g of an amylolytic enzyme, 2 g of a pectin decomposition enzyme, and 1,500 ml of water and the resultant mixture was allowed to stand at 50°C for 20 hours. Thereafter, the mixture was squeezed by a squeezer to provide 1,420 ml of the product of the present invention and 560 g of residues.

### Example 23

A further 1,440 ml of product of the present invention was obtained by following the same procedure as in Example 22 using 500 g of the product obtained in Example 1.

### Comparative Example 24

By following the same procedure as in Example 22, 2,000 g of the enzyme decomposition product of polished rice was obtained. Thereafter, the temperature thereof was gradually raised and after extracting under boiling for 5 minutes, the extract was cooled. Thereafter, the extract was squeezed by a squeezer to provide 1,400 ml of the product of the present invention and 550 g of residues.

### Example 25

A further 1,420 ml of product of the present invention was obtained by following the same procedure as in Example 24 using 500 g of the product obtained in Example 1.

### Comparative Example 26

Polished rice was ground in a grinder to provide 500 g of the ground product. To the ground product were added 300 g of 'koji' and 1,500 ml of 40% ethanol and the resultant mixture was allowed to stand at 55°C for 48 hours. Thereafter, the mixture was squeezed by a squeezer to provide 1,300 ml of a clear liquid and 850 g of residues. Then, to the clear liquid was added 1,000 ml of water and the mixture was concentrated by a rotary evaporator to provide 1,300 ml of the product of the present invention.

### Example 27

A further 1,300 ml of product of the present invention was obtained by following the same procedure as in Example 26 using 500 g of the product obtained in Example 1.

### Comparative Example 28

By following the same procedure as in Example 4, 2,000 g of the extract of polished rice was obtained. To the extract were added 2 g of a proteolytic enzyme, 2 g of lipase, 2 g of a fibre decomposition enzyme, 2 g of an amylolytic enzyme, and 2 of a pectin decomposition enzyme and the resultant mixture was allowed to stand at 50°C for 24 hours. Thereafter, the mixture was squeezed by a squeezer to provide 1,400 ml of the product and 580 g of residues.

### Example 29

A further 1,390 ml of product of the present invention was obtained by following the same procedure as in Example 28 using 500 g of the product obtained in Example 1.

### Comparative Example 30

By following the same procedure as in Example 24, 2,000 g of the enzyme decomposition extract of polished rice was obtained. To the enzyme decomposition extract was added yeast and an alcohol fermentation was carried out. Thereafter, the fermentation product was squeezed in a squeezer to provide 1,880 ml of the product of the present invention and 80 g of residues.

### Example 31

A further 1,800 ml of product of the present invention was obtained by following the same procedure as in Example 30 using 500 g of the product obtained in Example 1.

### Comparative Example 32

By following the same procedure as in Example 24, 2,000 g of the enzyme decomposition extract of polished rice was obtained. After sterilizing the enzyme decomposition extract by boiling, the extract was cooled to 37°C, and 200 ml of a starter previously prepared by culturing a lactic acid bacteria was added thereto followed by stirring well. Thereafter, the mixture was sealed tightly and the lactic acid fermentation was carried out at 37°C for 2 days. Then, the fermentation product was squeezed in a squeezer to provide 1,380 ml of the product and 590 g of residues.

### Example 33

A further 1,400 ml of product of the present invention was obtained by following the same procedure as in Example 32 using 500 g of the product obtained in Example 1.

### Comparative Example 34

To 1,000 ml of the product of this invention obtained in Example 24 was added 80 ml of 95% ethanol and the acetic acid fermentation was carried out for 20 hours. Thereafter, the fermentation product was filtrated to provide 990 ml of the product of the present invention.

### Example 35

A further 1,000 ml of product of the present invention was obtained by following the same procedure as in Example 34 using 500 g of the product obtained in Example 1.

Next, Examples of the preparation of tablets by compounding the product of the present invention, and the preparation of a soft drink using the product of the present invention, are described below. The present invention is not limited to the compounding Examples.

### Comparative Example 36 (Tablet)

By drying 100 g of the product obtained in Example 24 by freeze-drying, 20 g of the dried product was obtained. By using 10 g of the dried product, tablets were prepared as described below.

| | |
|---|---|
| Product of the invention | 10 g |
| Polyethylene glycol 6000 | 10 g |
| Sodium laurylsulfate | 1.5 g |
| Corn starch | 3 g |
| Milk sugar | 25 g |
| Magnesium stearate | 0.5 g |

After weighing each component described above, polyethylene glycol 6000 was heated to a temperature of from 70°C to 80°C and after mixing it with the product of this invention, sodium laurylsulfate, corn starch, and milk sugar, the mixture was cooled as it was. The solidified mixture was granulated in a grinder. Then, after mixing the granules with magnesium stearate, the mixture was tableted to form tablets each having a weight of 250 mg.

### Comparative Example 37 (Soft Drink)

In this Example, the product of this invention obtained in Example 22 was used.

| | |
|---|---|
| Product of the invention | 15 % by weight |
| Licorice extract | 0.01% by weight |
| Sugar | 4 % by weight |
| Lemon juice | 2.5% by weight |
| Purified water | 78.49% by weight |

By mixing the above components in an ordinary manner, a soft drink was obtained.

As described above in detail, each of the products of the present invention shows a remarkable effect with respect to digestive ulcers. The fact that the products of the present invention exhibit great effect via both oral and subcutaneous administration shows that the products can be practically used both as internal medicines as well as injections, and so widespread use of the product is expected. It is epoch-making that the products having such a remarkable anti-ulcer activity can be easily obtained at low cost from rice, the safety of which has been proved.

Furthermore, the products of the present invention are effective not only in the treatment of ulcers but also for their prophylaxis, since no problems are encountered even when the products are regularly used. Thus, the products of the present invention represent a significant advance in preventative medicine, and are valuable for preventing the recurrence of ulcers in previous sufferers.

## Claims

1. A composition comprising germinated rice, or an extract of germinated rice, for use in medicine.

2. A composition comprising germinated rice, or an extract of germinated rice, for use in the prophylaxis or treatment of ulcers.

3. A composition as claimed in Claim 1 or 2, wherein said germinated rice is in ground or particulate form, or said extract is derived from germinated rice in ground or particulate form.

4. A composition as claimed in Claim 1, 2 or 3, wherein the extract is obtained by hydrating germinated rice, allowing to stand at a temperature between room temperature and 40°C, and separating the liquid product.

5. A composition as claimed in Claim 1 or 2, wherein the extract is obtained by hydrating germinated rice, pre-treating a mixture of the hydrated product and water with amylase or 'koji', maintaining the mixture at a temperature of from 40°C to boiling, and separating the liquid product.

6. A composition as claimed in Claim 1 or 2, wherein the extract is obtained by treating germinated rice with either an acidic aqueous solution or an alkaline aqueous solution, allowing the mixture to stand for several days to form a liquid product, and separating the liquid product.

7. A composition as claimed in Claim 1 or 2, wherein the extract is obtained by hydrating germinated rice, subjecting the hydrated product to treatment with at least one enzyme selected from amylolytic enzymes, proteolytic enzymes, lipases, fibre decomposition enzymes, lignin decomposition enzymes, pectin decomposition enzymes and 'koji', and either separating the liquid product so obtained or boiling the mixture and then separating the liquid product.

8. A composition as claimed in Claim 1 or 2, wherein the extract is obtained by hydrating germinated rice, allowing the hydrated product to stand at a temperature of from room temperature to 40°C, subjecting the product to treatment with at least one enzyme selected from amylolytic enzymes, proteolytic enzymes, lipases, fibre decomposition enzymes, lignin decomposition enzymes, pectin decomposition enzymes and 'koji', and separating the liquid product.

9. A composition as claimed in Claim 1 or 2, wherein the extract is obtained by treating germinated rice with an organic solvent, and separating the liquid product.

10. A composition as claimed in Claim 1 or 2, wherein the extract is obtained by treating germinated rice with an organic solvent, subjecting the mixture so formed to treatment with at least one enzyme selected from amylolytic enzymes, proteolytic enzymes, lipases, fibre decomposition enzymes, lignin decomposition enzymes, pectin decomposition enzymes and 'koji', and separating the liquid product.

11. A composition as claimed in any one of Claims 3 to 10, wherein the liquid product is subjected to an alcohol fermentation or an organic acid fermentation.

12. The use, in the manufacture of a medicament for the prophylaxis or treatment of ulcers, of a composition as claimed in any preceding Claim.

## Patentansprüche

1. Zusammensetzung zur Verwendung in der Medizin, welche gekeimten Reis oder einen Extrakt von gekeimtem Reis umfaßt.

2. Zusammensetzung zur Verwendung für die Prophylaxe oder für die Behandlung von Geschwüren, welche gekeimten Reis oder einen Extrakt von gekeimtem Reis umfaßt.

3. Zusammensetzung nach Anspruch 1 oder 2, bei welcher der gekeimte Reis in gemahlener Form oder in Teilchenform vorliegt oder der Extrakt aus gekeimtem Reis in gemahlener Form oder in Teilchenform gewonnen wird.

4. Zusammensetzung nach den Ansprüchen 1, 2 oder 3, bei welcher der Extrakt erhalten wird, indem man gekeimten Reis hydriert, bei einer Temperatur zwischen Raumtemperatur und 40 °C stehen läßt und das flüssige Produkt abtrennt.

5. Zusammensetzung nach Anspruch 1 oder 2, bei welcher der Extrakt erhalten wird, indem man gekeimten Reis hydriert, ein Gemisch aus dem hydrierten Produkt und Wasser mit Amylase oder "koji" vorbehandelt, das Gemisch bei einer Temperatur zwischen 40 °C und Kochen beläßt und das flüssige Produkt abtrennt.

6. Zusammensetzung nach Anspruch 1 oder 2, bei welcher der Extrakt erhalten wird, indem man gekeimten Reis entweder mit einer sauren wäßrigen Lösung oder einer alkalischen wäßrigen Lösung behandelt, das Gemisch einige Tage lang stehen läßt, um ein flüssiges Produkt zu erhalten, und das flüssige Produkt abtrennt.

7. Zusammensetzung nach Anspruch 1 oder 2, bei welcher der Extrakt erhalten wird, indem man gekeimten Reis hydriert, das hydrierte Produkt einer Behandlung mit mindestens einem Enzym unterzieht, das aus amylolytischen Enzymen, proteolytischen Enzymen, Lipasen, Faser-abbauenden Enzymen, Lignin-abbauenden Enzymen, Pektin-abbauenden Enzymen und "koji" ausgewählt ist, und entweder das so erhaltene flüssige Produkt abtrennt oder das Gemisch kocht und nachfolgend das flüssige Produkt abtrennt.

8. Zusammensetzung nach Anspruch 1 oder 2, bei welcher der Extrakt erhalten wird, indem man gekeimten Reis hydriert, das hydrierte Produkt bei einer Temperatur zwischen Raumtemperatur und 40 °C stehen läßt, das Produkt einer Behandlung mit mindestens einem Enzym unterzieht, das aus amylolytischen Enzymen, proteolytischen Enzymen, Lipasen, Faserabbauenden Enzymen, Lignin-abbauenden Enzymen, Pektin-abbauenden Enzymen und "koji" ausgewählt ist, und das flüssige Produkt abtrennt.

9. Zusammensetzung nach Anspruch 1 oder 2, bei welcher der Extrakt erhalten wird, indem man gekeimten Reis mit einem organischen Lösungsmittel behandelt und das flüssige Produkt abtrennt.

10. Zusammensetzung nach Anspruch 1 oder 2, bei welcher der Extrakt erhalten wird, indem man gekeimten Reis mit einem organischen Lösungsmittel behandelt, das so gebildete Gemisch einer Behandlung mit mindestens einem Enzym unterzieht, das aus amylolytischen Enzymen, proteolytischen Enzymen, Lipasen, Faser-abbauenden Enzymen, Lignin-abbauenden Enzymen, Pektin-abbauenden Enzymen und "koji" ausgewählt ist, und das flüssigen Produkt abtrennt.

11. Zusammensetzung nach einem der Ansprüche 3 bis 10, bei welcher man das flüssige Produkt einer alkoholischen Fermentation oder einer organischen Säurefermentation unterzieht.

12. Verwendung einer Zusammensetzung gemäß eines vorhergehenden Anspruchs zur Herstellung eines Medikamentes zur Prophylaxe oder zur Behandlung von Geschwüren.

## Revendications

1. Composition comprenant du riz germé ou un extrait de riz germé pour une utilisation en médecine.

2. Composition comprenant du riz germé ou un extrait de riz germé pour une utilisation pour la prophylaxie ou le traitement d'ulcères.

3. Composition selon la revendication 1 ou 2, dans laquelle ledit riz germé est sous une forme broyée ou particulaire, ou ledit extrait provient de riz germé sous une forme broyée ou particulaire.

4. Composition selon la revendication 1, 2 ou 3, dans laquelle on obtient l'extrait en hydratant du riz germé, en le laissant reposer à une température comprise entre la température ambiante et 40°C et en séparant le produit liquide.

5. Composition selon la revendication 1 ou 2, dans laquelle on obtient l'extrait en hydratant du riz germé, en traitant préalablement un mélange du produit hydraté et d'eau avec une amylase ou du 'koji', en maintenant le mélange à une température comprise entre 40°C et le point d'ébullition et en séparant le produit liquide.

6. Composition selon la revendication 1 ou 2, dans laquelle on obtient l'extrait en traitant du riz germé avec une solution aqueuse acide ou une solution aqueuse alcaline, en laissant reposer le mélange pendant plusieurs jours pour former un produit liquide et en séparant le produit liquide.

7. Composition selon la revendication 1 ou 2, dans laquelle on obtient l'extrait en hydratant du riz germé, en soumettant le produit hydraté à un traitement avec au moins une enzyme choisie parmi des enzymes amylolytiques, des enzymes protéolytiques, des lipases, des enzymes de décomposition de fibres, des enzymes de décomposition de lignine, des enzymes de décomposition de pectine et 'koji', et en séparant le produit liquide ainsi obtenu ou en portant le mélange à ébullition et ensuite, en séparant le produit liquide.

8. Composition selon la revendication 1 ou 2, dans laquelle on obtient l'extrait en hydratant du riz germé, en laissant reposer le produit hydraté à une température comprise entre la température ambiante et 40°C, en soumettant le produit à un traitement avec au moins une enzyme choisie parmi des enzymes amylolytiques, des enzymes protéolytiques, des lipases, des enzymes de décomposition de fibres, des enzymes de décomposition de lignine, des enzymes de décomposition de pectine et 'koji', et en séparant le produit liquide.

9. Composition selon la revendication 1 ou 2, dans laquelle on obtient l'extrait en traitant du riz germé avec un solvant organique et en séparant le produit liquide.

10. Composition selon la revendication 1 ou 2, dans laquelle on obtient l'extrait en traitant du riz germé avec un solvant organique, en soumettant le mélange ainsi formé à un traitement avec au moins une enzyme choisie parmi des enzymes amylolytiques, des enzymes protéolytiques, des lipases, des enzymes de décomposition de fibres, des enzymes de décomposition de lignine, des enzymes de décomposition de pectine et 'koji', et en séparant le produit liquide.

11. Composition selon l'une quelconque des revendications 3 à 10, dans laquelle on soumet le produit liquide à une fermentation alcoolique ou à une fermentation avec un acide organique.

12. Utilisation d'une composition selon l'une quelconque des revendications précédentes pour la fabrication d'un médicament pour la prophylaxie ou le traitement d'ulcères.
